# FASCICULE DE BREVET EUROPEEN

(11) **EP 0 532 421 B1**
(45) Date de publication et mention de la délivrance du brevet: **08.11.1995**
(21) Numéro de dépôt: 92402485.4
(22) Date de dépôt: 11.09.1992
(51) Int. Cl.: A61F 2/30, A61B 17/58

(54) **Matériel d'ostéosynthèse à face de contact osseux recouverte par un revêtement de surface**
Osteosynthetische Gegenstände mit oberflächenbeschichteten Knochenberührungsflächen
Osteosynthetic devices with surface coated bone contact faces

(30) Priorité: 13.09.1991 FR 9111582
(43) Date de publication de la demande: 17.03.1993
(73) Titulaire: ITAC VAR-IMPLANT SARL, F-83200 Toulon (FR); Meyrueis, Jean Paul, F-83200 Toulon (FR)
(72) Inventeur: Meyrueis, Jean-Paul, F-83200 Toulon (FR); Meyrueis, Philippe, F-83200 Toulon (FR); Meyrueis, Jacques, F-83200 Toulon (FR)
(74) Mandataire: Cournarie, Michèle

(56) Documents cités:
- EP-A- 0 285 826
- EP-A- 0 340 174
- EP-A- 0 360 139
- EP-A- 0 413 492
- WO-A-81/02668
- WO-A-83/03346
- WO-A-85/05027
- WO-A-88/07355
- GB-A- 1 603 453
- US-A- 4 126 924
- US-A- 4 988 362

## Description

La présente invention est relative à des perfectionnements apportés aux plaques d'ostéosynthèse et aux clous destinés au traitement des fractures ou ostéotomies.

L'ostéosynthèse des os se fait actuellement par des plaques de métal, lisses ou rugueuses, qui rapprochent les fragments osseux et les maintiennent en contact. Le but de cette technique est de permettre la consolidation de la fracture par repousse osseuse sous l'implant.

On sait, par le brevet FR 7606753, que la rugosité de la face osseuse de la plaque améliore la qualité de la fixation mécanique en diminuant considérablement les contraintes, en cisaillement qui s'exercent sur les vis.

Le brevet GB-A-1 603 453 décrit des moyens de fixation utilisables en ostéosynthèse, ces moyens étant constitués d'une matière non conductrice d'électricité ou d'une matière conductrice d'électricité, agencée de telle manière que la distribution de biopotentiels dans l'os soumis à la fixation corresponde à la distribution naturelle de biopotentials dans l'os en l'absence de toute fracture. Le document EP-A-0 360 139 décrit une plaque d'ostéosynthèse en polymères biorésorbables. Ce document prévoit de revêtir la face de contact osseux du matériel par des petites particules de phosphate de calcium et/ou d'hydroxyapatite.

A l'heure actuelle, les plaques d'ostéosynthèse sont en général en acier inoxydable, en titane, ou en alliage de titane. Il se cree, avec ces métaux, une couche fibreuse entre l'os et la plaque.

Le besoin se fait donc sentir de mettre à disposition un revêtement de surface sur la face de la plaque en contact avec l'os, ameliorant la qualité du contact os-plaque, ou même, suivant la nature du revêtement, stimulant l'ostéogénèse.

A cet effet, l'invention a pour objet un matériel d'ostéosynthèse destiné au traitement chirurgical des fractures et aux ostéotomies, présentant une face de contact avec l'os, recouverte d'un revêtement de surface, caractérisé en ce que ladite face de contact, lisse ou rugueuse, est recouverte d'un revêtement de surface destiné à améliorer la qualité histologique du contact os-matériel, et à stimuler biologiquement l'ostéogénèse, ce revêtement de surface étant essentiellement constitué de biomatériaux actifs résorbables consistant en tricalcium phosphate, ou de biomatériaux actifs non résorbables consistant en hydroxyapatite, ou de biomatériaux inertes consistant en alumines et/ou carbones amorphes, ou de composés biologiques consistant en poudres de corail et/ou poudres d'os et/ou de coquilles, ou encore d'un mélange de plusieurs quelconques de ces produits, en une seule couche ou en plusieurs couches de même nature au de natures différentes, le substrat de ce matériel n'étant pas résorbable.

Selon l'invention, on obtient une stimulation de l'ostéogénèse qui accélère la consolidation de l'os et diminue le risque de pseudarthroses, c'est-à-dire de non-consolidation.

Les revêtements de biomatériaux résorbables tels que le tricalcium phosphate, sont particulièrement intéressants, car a l'instar des revêtements d'hydroxyapatites ils stimulent l'ostéogénèse, et de plus, se résorbent ensuite progressivement.

Le matériel selon l'invention est de préférence constitué d'une plaque, d'un clou, ou d'un clou-plaque et d'une vis-plaque. Lorsque ce matériel est un clou, le revêtement peut être appliqué sur la totalité, ou sur une partie de la face externe du clou, qui est en contact avec les parois du canal médullaire. Il est à noter que le substrat du revêtement selon l'invention est réalisé dans tous les métaux actuellement utilisés pour les plaques ou les clous tels qu'aciers inoxydables austénitiques, titane, alliages de titane, chromecobalt, les carbones, les céramiques, ainsi que tous les alliages des produits ci-dessus.

D'autre part, avant l'application du revêtement sur le substrat, la surface de ce dernier peut être lisse ou rugueuse.

La rugosité peut être obtenue par apport de métal poreux, par métallisation au chalumeau ou par frittage, par emboutissage à la presse, et plus largement par tout moyen mécanique avec ou sans enlèvement de matière, par électroérosion ou sablage, par apport de substance poreuse, notamment de céramique, par des méthodes physiques ou physico-chimiques. La rugosité est obtenue à l'état brut de fonderie ou à l'état brut de forge ou ultérieuremnt.

La surface peut avoir subi un traitement de nitruration ionique, d'implantation ionique, ou de dépôt de nitrure de titane.

Pour un bon accrochage des revêtements de surface, il est préférable de traiter la surface de l'implant, qu'elle soit lisse ou qu'elle comporte des macroaspérités, par apport de métal poreux ou sablage.

Le revêtement selon l'invention peut être déposé par mise en oeuvre de toutes techniques appropriées, notamment par tous procédés physiques de dépôt en phase vapeur, tels qu'évaporation, pulvérisation, évaporation et pulvérisation réactives, dépôts ioniques, ou électrophorèse, ou encore par tous procédés chimiques de dépôt en phase vapeur, statiques, tels que cémentation, dynamiques, ou dynamiques assistés tels que protection plasma et projection laser.

Des exemples de mise en oeuvre de l'invention vont maintenant être décrits en regard des dessins annexés, sur lesquels :
la figure 1 représente une plaque d'osthéosynthèse,
la figure 2 représente une plaque rugueuse en coupe transversale,
la figure 3 représente un détail de la figure 2,
la figure 4 représente une plaque lisse en coupe transversale,
la figure 5 représente un clou-plaque pour traitement des fractures du col du fémur, et
la figure 6 représente un clou pour ostéosynthèse avec revêtement de surface.

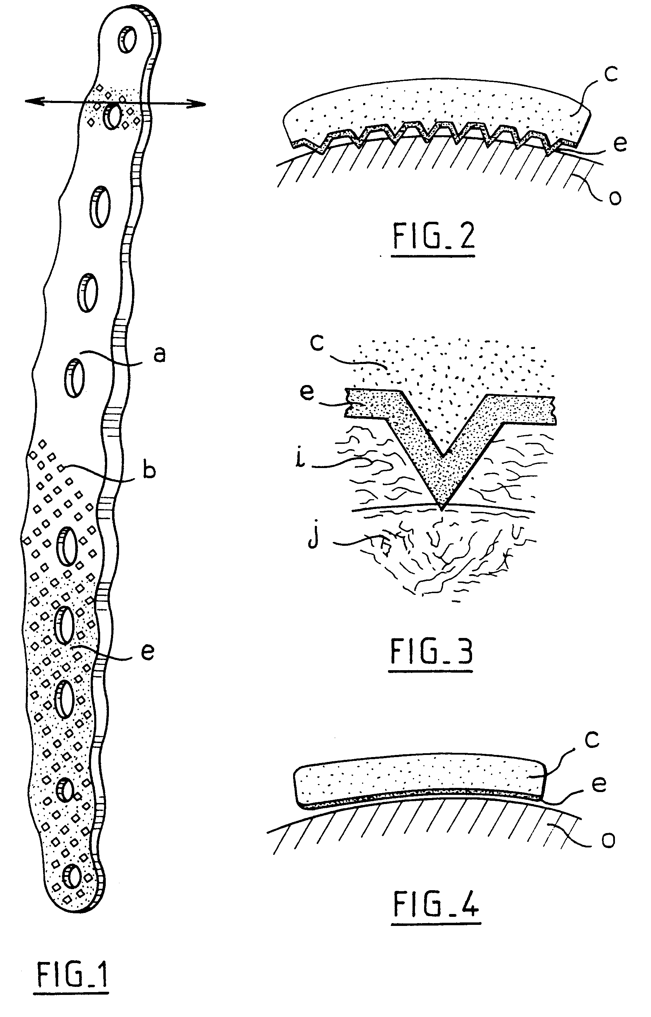

La plaque de la figure 1 comporte une face de contact osseux a, des aspérités ou rugosités b, un revêtement de surface e.

La plaque rugueuse c de la figure 2 est ancrée dans l'os o au moyen de rugosités en forme de chevrons recouverts d'un revêtement de surface e.

Sur la figure 3, on distingue la plaque c, dont une surface présentant un profil en chevrons est munie d'un revêtement de surface e en contact avec l'os néoformé i recouvrant l'os ancien j.

Sur la figure 4, la plaque lisse c comporte un revêtement de surface e en contact avec l'os o.

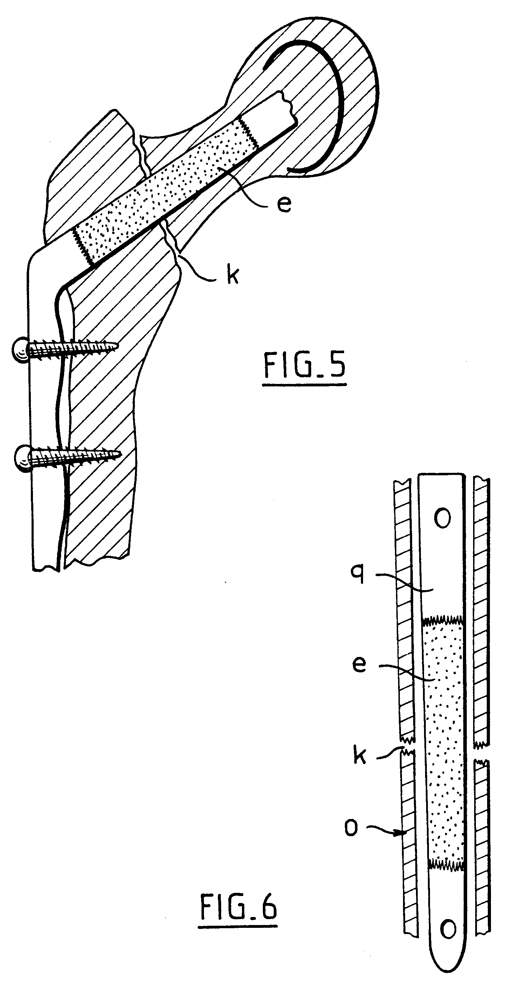

Sur la figure 5, un clou-plaque comporte un revêtement de surface e au voisinage d'une fracture k.

Sur la figure 6, un clou q comporte un revêtement de surface e au voisinage d'une fracture k de l'os o.

## Revendications

1. Matériel d'ostéosynthèse destiné au traitement chirurgical des fractures et aux ostéotomies, présentant une face de contact (a) avec l'os (o), recouverte d'un revêtement de surface (e) caractérisé en ce que la face de contact (a), lisse ou rugueuse, est recouverte d'un revêtement de surface (e) destiné à améliorer la qualité histologique du contact os-matériel, et à stimuler biologiquement l'ostéogénèse, ce revêtement de surface (e) étant essentiellement constitué de biomatériaux actifs résorbables ou de biomatériaux actifs non résorbables, consistant respectivement en les phosphates de calcium tricalcium phosphate et hydroxyapatite, au de biomatériaux inertes consistant en alumines et/ou en carbones amorphes, ou de composés biologiques consistant en poudres d'os et/ou de corail et/ou de coquille, ou encore d'un mélange de plusieurs quelconques de ces produits, le substrat de ce matériel n'étant pas résorbable.

2. Matériel selon la revendication 1, caractérisé en ce que le revêtement de surface (e) consiste en une seule couche ou en plusieurs couches de même nature ou de natures différentes.

3. Matériel suivant la revendication 1 ou 2, caractérisé en ce qu'il est constitué d'une plaque (c).

4. Matériel suivant la revendication 1 ou 2, caractérisé en ce qu'il est constitué d'un clou (q).

5. Matériel suivant la revendication 1 ou 2, caractérisé en ce qu'il est constitué d'un clou-plaque et d'une vis-plaque.

## Claims

1. An osteosynthesis material for surgical treatment of fractures and of osteotomies, having a contact face (a) with the bone (o) that is covered with a surface coating (e), the material being characterized in that the smooth or rough contact face (a) is covered with a surface coating (e) for improving the histological quality of bone/material contact and for biologically stimulating osteogenesis, said surface coating (e) being essentially constituted by resorbable active biomaterials or by non-resorbable active biomaterials, respectively consisting of the calcium phosphates: tricalcium phosphate and hydroxyapatite, or by inert biomaterials consisting of aluminas and/or amorphous carbons, or by biological compounds consisting of powders of bone and/or of coral and/or of shell, or else by a mixture of any selection of said substances, the substrate of said material not being resorbable.

2. A material according to claim 1, characterized in that the surface coating (e) comprises a single layer or a plurality of layers of the same kind or of different kinds.

3. A material according to claim 1 or 2, characterized in that it is constituted by a plate (c).

4. A material according to claim 1 or 2, characterized in that it is constituted by a pin (q).

5. A material according to claim 1 or 2, characterized in that it constituted by a pin-plate and a screw-plate.

## Patentansprüche

1. Osteosynthesematerial für die chirurgische Behandlung von Frakturen und für Osteotomien, mit einer Kontaktfläche (a) mit dem Knochen (o), welche mit einer Oberflächenbeschichtung (e) überzogen ist, dadurch gekennzeichnet, daß die glatte oder rauhe Kontaktfläche (a) mit einer Oberflächenbeschichtung (e) überzogen ist, die dazu bestimmt ist, die histologische Eigenschaft des Kontaktes Knochen/Material zu verbessern und die Osteogenese biologisch anzuregen, wobei diese Oberflächenbeschichtung (e) im wesentlichen von resorbierbaren aktiven Biomaterialien oder nichtresorbierbaren aktiven Biomaterialien gebildet wird, welche aus den Calciumphosphaten Tricalciumphosphat bzw. Hydroxylapatit bestehen, oder von inerten Biomaterialien, welche aus Aluminiumoxiden und/oder amorphen Kohlenstoffen bestehen, oder von biologischen Zusammensetzungen, welche aus Knochen- und/oder Korallen- und/oder Muschelmehlen bestehen, oder noch von einer Mischung von mehreren beliebigen dieser Produkte, wobei das Substrat dieses Materials nicht resorbierbar ist.

2. Material nach Anspruch 1, dadurch gekennzeichnet, daß die Oberflächenbeschichtung (e) aus einer einzigen Schicht oder aus mehreren Schichten gleicher oder verschiedener Art besteht.

3. Material nach Anspruch 1 oder 2, dadurch gekennzeichnet, daß es von einer Platte (c) gebildet wird.

4. Material nach Anspruch 1 oder 2, dadurch gekennzeichnet, daß es von einem Nagel (q) gebildet wird.

5. Material nach Anspruch 1 oder 2, dadurch gekennzeichnet, das es von einer Kombination aus Nagel und Platte oder einer Kombination aus Schraube und Platte gebildet wird.
